(19) **Europäisches Patentamt / European Patent Office / Office européen des brevets**

(11) **EP 2 081 024 A1**

(12) **EUROPEAN PATENT APPLICATION**
published in accordance with Art. 153(4) EPC

(43) Date of publication:
**22.07.2009 Bulletin 2009/30**

(51) Int Cl.:
*G01N 33/483* (2006.01)    *G01N 15/14* (2006.01)
*G01N 33/579* (2006.01)

(21) Application number: **06810474.4**

(22) Date of filing: **25.09.2006**

(86) International application number:
**PCT/JP2006/318906**

(87) International publication number:
**WO 2008/038329 (03.04.2008 Gazette 2008/14)**

(84) Designated Contracting States:
**AT BE BG CH CY CZ DE DK EE ES FI FR GB GR HU IE IS IT LI LT LU LV MC NL PL PT RO SE SI SK TR**

(71) Applicants:
• **Kowa Kabushiki Kaisha**
**Nagoya-shi,**
**Aichi 460-8625 (JP)**
• **Obata, Toru**
**Suginami**
**Tokyo 168-0064 (JP)**

(72) Inventors:
• **OBATA, Toru**
**Tokyo 168-0064 (JP)**
• **SHIRASAWA, Yoshiaki**
**Chuo-ku**
**Tokyo 103-8433 (JP)**

(74) Representative: **Kronthaler, Wolfgang N.K.**
**Kronthaler, Schmidt & Coll.,**
**Steinsdorfstrasse 5**
**80538 München (DE)**

(54) **APPARATUS FOR GELATION MEASUREMENT AND SAMPLE CELL**

(57) It is intended to allow the concentration of a substance such as an endotoxin or β-D glucan measured via gelation reactions to be measured in a short time and at a high sensitivity and a high specificity. In a measuring apparatus for measuring a target substance in a sample cell via a gelation reaction, a sample cell 13 for housing a specimen containing the substance to be measured and a solution containing a gelating reagent is irradiated with a laser beam from a semiconductor laser 11. The solution in the sample cell is stirred by a stir bar 25 to generate minute and uniform gel particles, which are caused to pass through the laser beam. Scattered light from the gel particles generated in the sample cell 13 is detected by a photodiode array 18, and a scattered-light intensity of the generated gel particles or the diameter and the number thereof is measured on time series by a computer 21 on the basis of a scattered-light detection output of the photodiode array.

*FIG. 1*

EP 2 081 024 A1

## Description

Technical Field

The present invention relates to a gelation measuring apparatus for detecting the progression of gelation and thereby measuring the concentration of an measurement object such as endotoxin or β-D glucan in a sample, and relates to a sample cell.

Background Art

Endotoxins (intracellular toxins) are lipopolysaccharides (LPS) in which a lipid called lipid A among the lipopolysaccharides (macromolecular complexes of phospholipids and polysaccharides) that constitute the cell walls of Gram-negative bacteria is linked with polysaccharide chains via 2-keto-3-deoxyoctonate (KDO). Endotoxins are released only when the cell wall breaks due to cell death or the like. Endotoxins are toxic substances that exert a variety of effects on living organisms, and in particular cause fever or lethal septicemia or shock. Endotoxins are thought to be an inciting factor in DIC (disseminated intravascular coagulation). It is important that pharmaceuticals (injected agents and the like) and medical devices (angiocatheters and the like) are not contaminated with endotoxins (are pyrogen free), and endotoxins must be completely removed from pharmaceuticals (recombinant proteins, DNA used in gene therapy and the like) that are prepared using bacteria.

Speed is needed when confirming the removal of endotoxins or measuring endotoxins in emergency medicine, not only from the perspective of the number of measurement samples but for the purposes of life-saving medical care.

Levin and Bang in 1964 discovered that endotoxins cause components of the blood-cell extract of horseshoe crabs to coagulate (gelate). The endotoxin detecting method in which this phenomenon is used is called the limulus test. Well-known methods for measuring gelation phenomena further include gelation methods for measuring the concentration of endotoxins from the dilution factor of a gelating specimen solution, and nephelometric methods for measuring the concentration of endotoxins based on the change in turbidity due to the gelation reaction. Other well-known methods include, e.g., chromogenic synthetic substrate methods in which a chromogenic synthetic substrate (Boc-Leu-Bly-Arg-p-nitroanilide) is added to the coagulation process instead of a coagulation precursor substance (coagulogen) to liberate p-nitroanilide by hydrolysis of the substrate and measure the concentration of endotoxin colorimetrically using the yellow chromogenicity of p-nitroanilide.

Also well-known are configurations in which the progression of the reaction of the limulus reagent is measured optically; particularly, in which measurement is performed based on a nephelometric method for measuring the change in the amount of transmitted light due to the gelation reaction. Well-known configurations involve, e.g., measuring the change over time in the intensity of transmitted light in a solution in which the limulus reagent and a specimen are mixed. The concentration of endotoxin in the specimen is measured from the amount of change recorded within a predetermined time period (the following Patent Document 1).

Measurement technology employing the same gelation reaction is used for measuring not only endotoxins, but also, e.g., β-D glucans.

β-D glucans are polysaccharides that constitute the characteristic cell membranes of fungi. Measuring β-D glucans is effective for, e.g., screening for a wide variety of fungal infections including not only fungi commonly seen in general clinical settings, such as Candida, Aspergillus, and Cryptococcus, but also rare fungi.

The coagulation (gelation) of components of the blood-cell extract of horseshoe crabs due to β-D glucans is also used for measuring β-D glucans. Measurement is performed using the same gelation, nephelometric, and chromogenic synthetic substrate methods as described above.

The methods for measuring endotoxins and β-D glucans have common elements such as, e.g., the use of substantially identical hardware. Gelation or chromogenic reactions selective for endotoxins can be measured by removing the Factor G component from the blood-cell extract of the horseshoe crab, and gelation or chromogenic reations selective for β-D glucans can be performed by inactivating endotoxins in the sample by pretreatment.

Patent Document 1: JP-A-2004-93536

Disclosure of Invention

Problems to be Solved

The following problems are presented in conventional limulus tests.

Among the above-mentioned methods, a gelation method is a method in which a limulus reagent fluid is mixed with a sample and is left at a given temperature to measure the time until the generation of a gel having low fluidity.

In the same manner, a nephelometric method is a method in which the change in turbidity due to the gelation reaction is detected as the change in the amount of transmitted light, and the time from the initiation of the reaction until the amount of transmitted light reaches a set proportion is measured as the gelation time.

A long period of approximately 90 minutes is required for the generation of the gel in all of the above-mentioned methods. Specifically, the gelation time of the reaction fluid is proportional to the concentration of the substance to be measured. However, taking into account the sensitivity of gelation and nephelometric methods, the time of gelation initiation cannot be accurately detected. Therefore, reaction variables are calculated from the time until the completion of gelation to estimate the gelation time. Gelation and nephelometric methods are therefore not suitable in emergencies or when measuring large numbers of specimens. Non-specific turbidity unrelated to endotoxins may also occur in nephelometric methods, and measurement precision is adversely affected. The measurement limit for gelation methods is a concentration of 3 pg/ml, and the measurement limit for nephelometric methods is a concentration of approximately 1 pg/ml.

In comparison to gelation and nephelometric methods, chromogenic synthetic substrate methods have a short measurement time of 30 minutes, but false-positive reactions may occur, and performing measurements with high specificity is difficult. Measurement preparations are also cumbersome, and the measurement-limiting concentration of 3 pg/ml is inferior to nephelometric methods.

It is an object of the present invention to solve the above-mentioned problems and to be able to measure the concentration of substances measured via gelation, such as endotoxins, β-D glucans and the like, within a short time and at a high sensitivity and a high specificity.

Means for Solving the Problems

In order to solve the above-mentioned problems, the present invention provides a gelation-reaction measuring apparatus for measuring a target substance in a sample via a gelation reaction. The apparatus comprises:

a sample cell for housing a specimen containing the target substance to be measured and a solution containing a gelating reagent;
a means for irradiating the sample cell with a laser beam from a laser light source;
a means for stirring the solution in the sample cell to generate minute and uniform gel particles, which are caused to pass through the laser beam;
a photoreceptive element for receiving the laser beam of light scattered by the gel particles generated in the sample cell;
a measuring means for measuring the diameter of the gel particles and the number thereof on time series based on the output of the scattered light detection of the photoreceptive element; and
a means for displaying a scattered light intensity of the measured gel particles or the diameter and the number thereof.

A sample cell of the present invention comprises a container which is sealed by a sealing member and which previously contains therein a reagent that gelates with the target substance to be measured, and a stirring means for stirring an introduced sample and the solution of the reagent.

Effect of the Invention

According to the constitution as mentioned above, the laser beam of light scattered by the gel particles generated in the sample cell is received, and the diameters and numbers of the gel particles are measured on time series based on the output of the scattered light detection. Thus, the concentration of substances such as endotoxins or β-D glucans measured via a gelation reaction can be measured within a short time and at a high sensitivity and a high specificity in comparison with measurement systems based on conventional nephelometric methods using transmitted light.

The sample cell comprises a container which is sealed by a sealing member and which previously contains therein a reagent that gelates with the target substance to be measured, and a stirring means for stirring an introduced sample and the solution of the reagent. This makes it possible to reduce the possibility of erroneous measurement resulting from the invasion of the measurement substance into the sample during transport or handling, thus allowing the aforementioned highly sensitive measurements.

Brief Description of Drawings

FIG. 1 is an illustrative view showing the configuration of a measurement apparatus employing the present invention;

FIG. 2A is an illustrative view showing an example of measurement of endotoxin according to the measurement apparatus of FIG. 1;

FIG. 2B is an illustrative view showing an example of measurement of endotoxin according to the measurement apparatus of FIG. 1;

FIG. 2C is an illustrative view showing an example of measurement of endotoxin according to the measurement apparatus of FIG. 1;

FIG. 2D is an illustrative view showing an example of measurement of endotoxin according to the measurement apparatus of FIG. 1;

FIG. 3A is an illustrative view showing an example of measurement of endotoxin according to the measurement apparatus of FIG. 1;

FIG. 3B is an illustrative view showing an example of measurement of endotoxin according to the measurement apparatus of FIG. 1;

FIG. 3C is an illustrative view showing an example of measurement of endotoxin according to the measurement apparatus of FIG. 1;

FIG. 4 is an illustrative view showing the configuration of a scattered-light measuring system of the measurement apparatus of FIG. 1;

FIG. 5 is a waveform diagram showing a measurement signal obtained from the scattered-light measuring system of the measurement apparatus of FIG. 1;

FIG. 6 is an illustrative view showing the circuit of the scattered-light measuring system of the measurement apparatus of FIG. 1; and

FIG. 7 is an illustrative view showing the configuration of a sample cell of the measurement apparatus of FIG. 1.

Key to Symbols

| 11 | Semiconductor laser |
| 12 | Condensing lens |
| 13 | Sample cell |
| 14 | Light-emitting diode (LED) |
| 15 | Magnetic stirrer |
| 16 | Sample solution |
| 17 | Light-receiving lens |
| 18 | Photodiode array |
| 19 | Amplifier |
| 20 | A/D converter |
| 21 | Computer |
| 22 | Photodiode |
| 25 | Stir bar |
| 77 | Pinholes |
| 85 | Operational amplifier |
| 87 | Absolute-value circuit |
| 90_1, 90_2 ... 90_n | Window comparators |
| 91_1, 91_2 ... 91_n | Counters |
| 131 | Container |
| 132 | Sealing member |
| 133 | Limulus reagent |

Best Mode of Carrying Out the Invention

The best mode of carrying out the invention involves embodiments relating to a measurement apparatus in which a limulus reagent is used to detect a gelation reaction and thereby measure the concentration of an endotoxin.

Embodiment 1

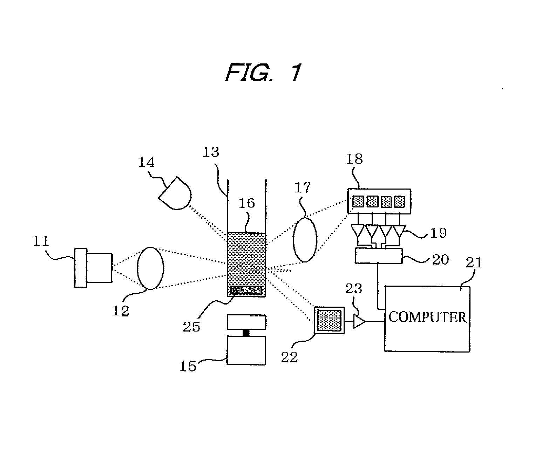

FIG. 1 shows the configuration of a measurement apparatus employing the present invention. In FIG. 1, a laser beam emitted from a semiconductor laser 11 for measuring the intensity of scattered light is collimated into sheet light by a condensing lens 12 and directed onto the vicinity of an inner wall inside a sample cell 13 (made of, e.g., glass).

Light from a light-emitting diode (LED) 14 is also directed onto the sample cell 13, and the transmitted light is received by a photodiode 22 in order to measure the transmitted light.

A sample solution 16 within the sample cell 13 is held at a constant temperature of 37°C, and rotated and stirred at 1000 rpm by a stir bar 25 and a magnetic stirrer 15 in order to generate minute and uniform gel particles.

Scattered light from the gel particles in the sample solution 16 is measured via a light-receiving lens 17 by a photodiode array 18, which is composed of a plurality of photodiodes as photoreceptor elements. The measurement results are output as an electrical signal.

The photodiodes of the photodiode array 18 are used which have light-receiving areas capable of receiving scattered light from observation regions in which only a single gel particle can be measured statistically. The output of the photodiode array 18 is subjected to current/voltage conversion by an amplifier 19, then subjected to A/D conversion by an A/D converter 20 and is input to a computer 21. Transmitted light detected by the photodiode 22 is also subjected to similar amplification and A/D conversion (not shown) and is input to the computer 21.

The scattered-light measurement signal that has been converted into a digital signal is subjected to signal processing by the computer 21, which is configured using, e.g., the hardware of a personal computer. The computer 21 includes operating devices such as a keyboard or mouse (not shown); a display for displaying measurement results; output devices such as a printer; and a network interface for inputting or outputting measurement results or information related to the measurements from or to other devices (not shown).

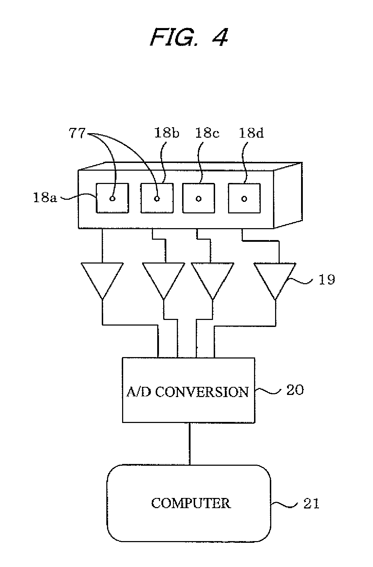

FIG. 4 shows in detail the configuration of a scattered-light measuring system of FIG. 1.

Scattered light from the sample solution is measured via the lens 17 as an electrical signal by a plurality of photodiodes 18a through 18d (photoreceptive elements) that constitute the photodiode array 18. Pinholes 77 are disposed on the forward parts of each of the photodiodes in order to receive scattered light from the observation regions in which only a single gel particle of the measurement object can be measured. The output of the photodiodes 18a through 18d is subjected to current/voltage conversion by the amplifier 19 and, after amplification, subjected to A/D conversion by the A/D converter 20 and is input to the computer 21.

The sizes of a single gel particle in the measurement object necessary for the determination of the sizes of the pinholes 77 can be statistically calculated and determined from measurement results obtained in advance.

In the computer 21, a plurality of comparators provided according to the diameters of the gel particles identifies the levels of the scattered-light intensity signals. The output signals from the comparators are counted by counters to measure the numbers of gel particles of predetermined diameters. Erroneously measured gel-particle diameters resulting from the passing of portions of gel particles through edge parts of the pinholes 77 are corrected by the measurement software of the computer that uses an equation derived from statistical probability theory and measurement results of standard particles.

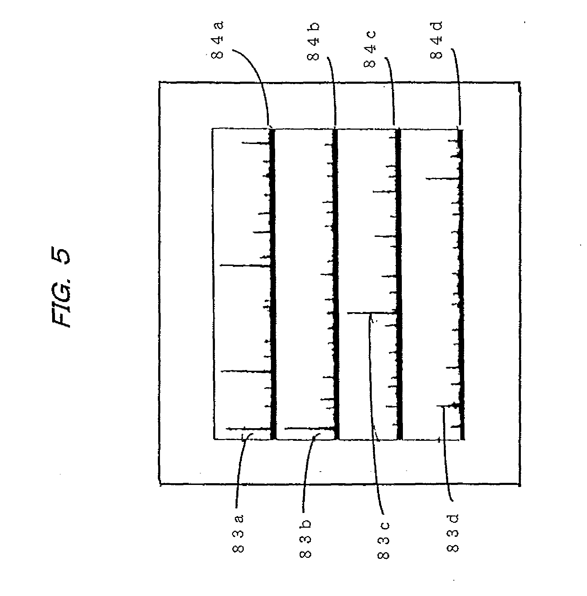

FIG. 5 shows how the scattered-light intensity signals measured by the photodiodes 18a through 18d of FIG. 4 change in the gelation-measuring process. The scattered light from the gel particles is measured as peak signals 83a through 83d, which correlate with the sizes of the particles, and the scattered light from other portions of the sample solution is measured as background signals 84a through 84d.

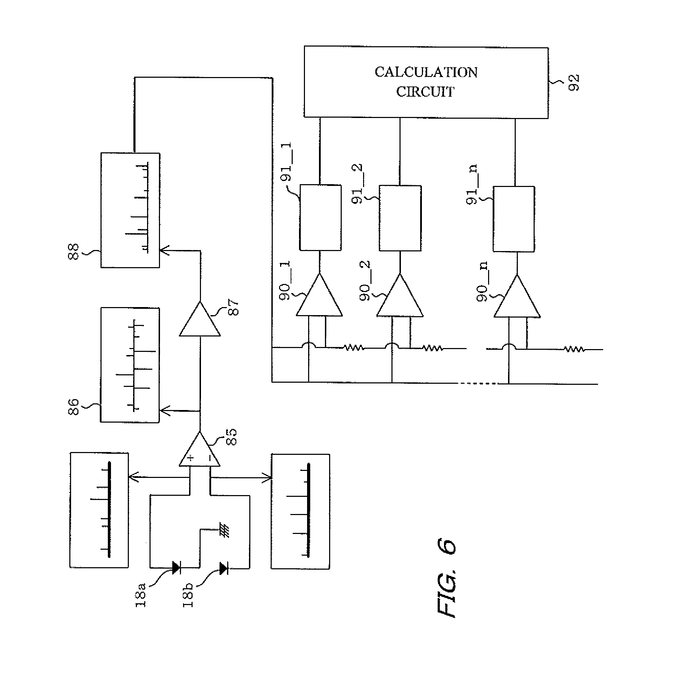

In order to negate the effect of the background signals, the respective output signals from the two photodiodes 18a, 18b (photoreceptive elements) are input to an operational amplifier 85 and subtracted, as shown in FIG. 6. This allows the background signals to be canceled out, and only the change in scattered-light intensity from the gel particles to be measured, as shown at 86. The signal from which the background signal has been removed is input to an absolute-value circuit 87. The output of the absolute-value circuit 87 becomes a signal containing only the peak signal without background, as shown at symbol 88.

The output signals from the absolute-value circuit are input to respective window comparators 90_1, 90_2 ... 90_n, to identify the levels of the signals. The comparators compare levels corresponding to the diameters of the gel particles. Therefore, each of the outputs of the comparators is a signal corresponding to each of the gel particle diameters. These signals are counted by respective counters 91_1, 91_2 ... 91_n to count the numbers of gel particles of the relevant diameters. The data resulting from the counting is input to a calculation circuit 92 (or directly to the computer 21) and is used in the process for measuring the gel particles (described hereinafter).

The computer 21 of FIG. 1 measures the scattered-light intensities of the gel particles or the diameters and numbers thereof and outputs them to a display. The computer further calculates a total scattering intensity Xt per unit time from the equation

$$X = \Sigma(\omega k \cdot Pk) \quad (1)$$

(where $\omega k$ is a weighting coefficient for particles having a scattering intensity Pk). The results are shown on a display (not shown).

A specimen containing a substance to be measured and a solution containing a gelating reagent are put into the sample cell 13. The computer calculates and displays the concentration of the target substance using a time TL (lag time) until a total scattering intensity Xt at or above a set level is measured after initiation of measurement, and the correlation between the TL and the amount (concentration) of the substance to be measured in the sample solution.

The computer further calculates and displays the concentration of the target substance using a maximum value Vmax of a generation velocity V (V = dXt/dt) of gel particles generated by the gelation reaction, and the correlation between the Vmax and the amount (concentration) of the substance to be measured in the sample solution.

The computer also calculates and displays the concentration of the target substance using the correlation between a maximum generated amount Xmax of gel particles generated by the gelation reaction and the amount (concentration) of the substance to be measured in the sample solution.

Examples of endotoxin measurements using a measurement apparatus in which the present invention is employed are given below.

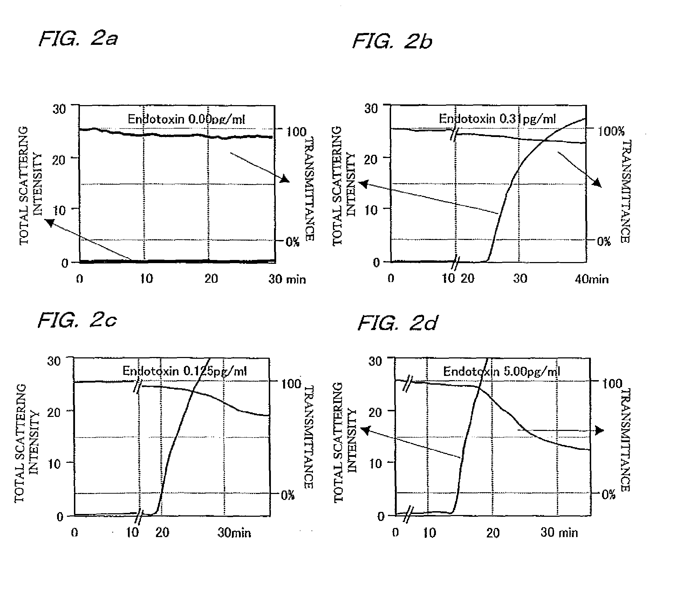

Measurement results of total scattering intensity and transmittance are shown for a sample containing 0.00 pg/ml endotoxin in FIG. 2A, for a sample containing 0.31 pg/ml endotoxin in FIG. 2B, for a sample containing 1.25 pg/ml endotoxin in FIG. 2C, and for a sample containing 5.00 pg/ml endotoxin in FIG. FIG 2D.

In these measurements, a limulus reagent and a sample containing endotoxin were introduced into the sample cell 13 of the apparatus of FIG. 1. Stirring was initiated, and measurement of scatted light was performed by the photodiode array 18, the amplifier 19, the A/D converter 20, and the computer 21. The transmitted light was also measured in the same samples using the light-emitting diode (LED) 14 and the photodiode 22 in order to validate the measurements of a conventional nephelometric method.

During measurement, the computer 21 displays time series changes in the total scattering intensity Xt of the gel particles due to the gelation reaction. The time TL until a total scattering intensity Xt at or above a set level is measured, and the maximum value Vmax of the generation velocity of the gel particles and the maximum generated amount Xmax of gel particles generated by the gelation reaction are calculated and displayed. The change in transmittance of the sample is also simultaneously measured, calculated, and displayed.

The transmittance gradually decreases in samples that do not contain endotoxin, as is seen in FIG. 2A. The conventional nephelometric methods for measuring transmittance therefore demonstrate the problem of detection of non-specific turbidity unrelated to endotoxin. On the other hand, the total scattered-light intensity measured from the sample of FIG. 2A using the present apparatus does not change, and it can be seen that non-specific turbidity is not measured by the laser particle scattering measurements of the present invention employing the total scattered-light intensity. In the measurements of the samples of FIGS. 2B, 2C, and 2D, non-specific turbidity as mentioned above is also detected by the decrease in transmittance when using the nephelometric method.

Commonly, a nephelometric method employs a method called a turbidimetric time assay, and the amount of endotoxin in the sample is measured using the correlation between the concentration of endotoxin and the time (gelation time) until the transmittance reaches a set value (the gelation determination threshold). The disadvantages of this method are that measurement of low concentrations of endotoxin is not possible when the gelation determination threshold is set low, and non-specific turbidity is mistakenly measured as endotoxin when the gelation determination threshold is set high, thus disadvantageously decreasing the measurement precision. In, e.g., FIG. 2B, determining the measurement value for the low endotoxin concentration of 0.31 pg/ml is not possible when the gelation determination threshold is set to 70% transmittance. Alternatively, determining the endotoxin concentration is possible when the measurement time until reaching a transmittance of 70% is extended, but the measurement requires a long time.

In, e.g., FIG. 2A, endotoxin of low concentrations can be measured in a short time when the gelation determination threshold is set to a large transmittance of 90%, but non-specific turbidity is mistakenly measured as endotoxin. The turbidimetric time assay can thus be regarded to involve a trade-off between precision and measurement time.

In contrast, the employment of a method for measuring the total scattered-light intensity as in the present invention ensures rapid and highly precise measurements of endotoxin without being affected by the occurrence of non-specific turbidity.

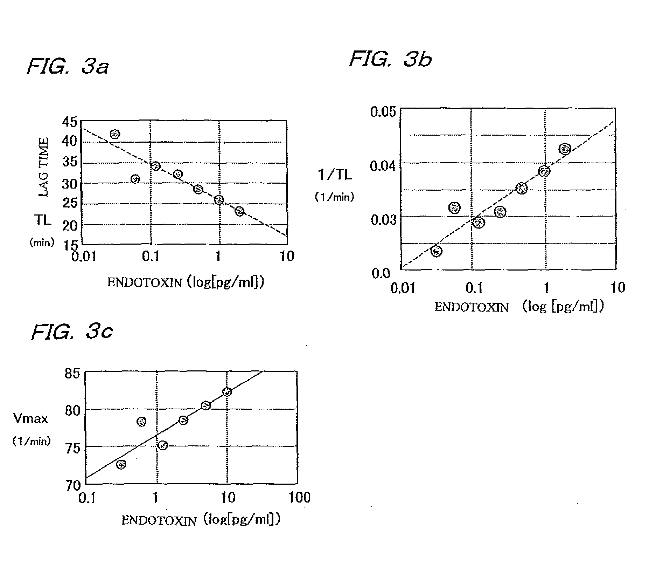

FIGS. 3A through 3C show measurement results for endotoxin concentration and the lag time TL of the total scattering intensity Xt, the reciprocal 1/TL and the maximum generation velocity Vmax that are calculated and displayed in the above-mentioned endotoxin measurements.

In FIG. 3A, the relationship between the lag time TL and endotoxin concentrations of 0.031 pg/ml, 0.063 pg/ml, 0.125 pg/ml, 0.25 pg/ml, 0.50 pg/ml, 1.0 pg/ml, and 2.0 pg/ml is linear with a negative slope.

As shown in FIG. 3B, the relationship between the reciprocal 1/TL of the lag time and the endotoxin concentration is linear with a positive slope. In FIG. 3C, the relationship between the maximum generation velocity Vmax and the endotoxin concentrations of 0.31 pg/ml, 0.63 pg/ml, 1.25 pg/ml, 2.5 pg/ml, 5.0 pg/ml, and 10 pg/ml is linear with a negative slope.

As described above, an extremely low endotoxin concentration of 0.031 pg/ml can be measured using the measurement apparatus of the present invention. The measurement apparatus of the present invention also makes possible measurements that are ten times more sensitive than conventional nephelometric methods that have a minimum measurement sensitivity of 0.3 pg/ml. The measurement time for 0.3 pg/ml of endotoxin is approximately 30 minutes using the measurement apparatus of the present invention, and measurements can be made in a short time that is one third that of conventional nephelometric measurements. In other words, the endotoxin concentration measured via a gelation reaction can be measured in a short time with high sensitivity and specificity according to the measurement technique of the present invention.

The description was made with reference to the embodiment in which both the limulus reagent and the sample were introduced in the sample cell 13.

However, the measurement sensitivity in the present invention as described above is high; i.e., approximately 10 times that of the prior art, and therefore it would be necessary that the structure surrounding the sample cell must be designed to be endotoxin-free.

Specifically, endotoxins are present in significant amounts in normal environments, and it is possible that some amount of endotoxin makes the sample cell impure during a reagent-manufacturing step or during the measurement operation.

The prior art is endotoxin-free at such a level that one end of the sample cell 13 is open or is able to be opened and closed in order to allow input of both the limulus reagent and the sample. However, the measurement apparatus of the present invention may positively detect endotoxin even for an endotoxin-free sample due to influences of invading endotoxin.

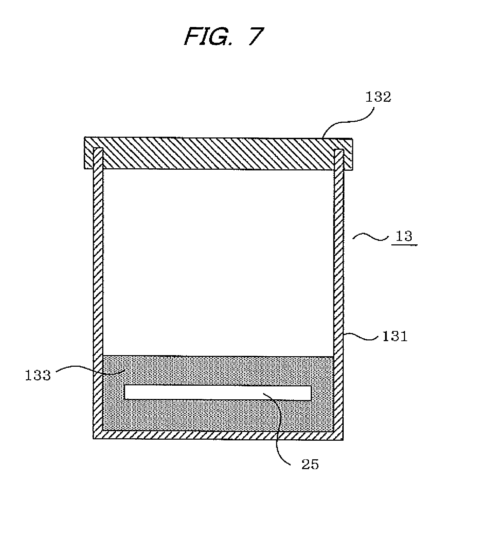

Accordingly, the sample cell 13 can be constructed with the stir bar 25 and a necessary amount of a limulus reagent 133 housed in a container 131 composed of resin, glass, or the like, and the upper part is sealed with a sealing member 132, as shown in FIG. 7.

The sealing member 132 may be in any desired form, but it shall be apparent that a member is used which has specifications such that endotoxin does not invade during the process of transport and handling.

The introduction of a measurement sample (specimen) into the sample cell 13 may be performed such that an injection needle or the like is used to puncture the sealing member 132 and perform an injection. Alternatively, in order to facilitate the introduction of the measurement sample (specimen), the sealing specifications of the sealing member 132 may also be set such that the interior of the sample cell 13 is maintained at a set negative pressure relative to atmospheric pressure.

It shall be apparent that the sample cell 13 as shown in FIG. 7 must be assembled in a manufacturing environment that achieves a set endotoxin-free level.

The sample cell 13 configured as shown in FIG. 7 can be supplied to a user in the form of an accessory to the measurement apparatus shown in FIGS. 1, 4, 5 and 6; or e.g., as a measurement kit constituting part of a product family. A measurement environment meeting a predetermined endotoxin-free level can be readily created in such instances, and highly precise measurement results can be reliably achieved.

FIG. 7 shows the configuration of a sample cell for a single specimen (sample), but a plurality of similar structures is integrated to provide a product that allows multiple specimens (samples) to be measured simultaneously and easily. It shall be apparent that a plurality of measurement apparatuses as shown in FIG. 1 must also be provided corresponding to the number of sample cells.

Endotoxins were assumed as the substance to be measured in the above-mentioned embodiment, but it shall be apparent that the same measurement hardware can be applied to similar measurements for detecting the progression of gelation phenomena for β-D glucans and the like using the same or similar limulus reagents.

Industrial Applicability

The present invention can be carried out in a variety of measurement apparatuses for detecting the progression of gelation phenomena and thereby measuring the concentration of measurement objects such as endotoxins or β-D glucans in a sample using a limulus reagent.

**Claims**

1.  A gelation-reaction measuring apparatus for measuring a target substance in a sample via a gelation reaction, comprising:

    a sample cell for housing a specimen containing the target substance to be measured and a solution containing a gelating reagent;
    a means for irradiating the sample cell with a laser beam from a laser light source;
    a means for stirring the solution in the sample cell to generate minute and uniform gel particles, which are caused to pass through the laser beam;
a photoreceptive element for receiving the laser beam of light scattered by the gel particles generated in the sample cell;
a measuring means for measuring the diameter of the gel particles and the number thereof on time series based on the output of the scattered light detection of the photoreceptive element; and
a means for displaying a scattered light intensity of the measured gel particles or the diameter and the number thereof.

2. A gelation-reaction measuring apparatus according to claim 1, wherein the photoreceptive element is configured so as to receive the scattered light from approximately one measurement particle.

3. A gelation-reaction measuring apparatus according to claim 1 or 2, wherein a plurality of the photoreceptive elements are provided, and scattered light corresponding to the photoreceptive elements is measured simultaneously.

4. A gelation-reaction measuring apparatus according to claim 3, wherein outputs from a pair of the photoreceptive elements are subtracted to increase a proportion of an effective signal.

5. A gelation-reaction measuring apparatus according to any one of claims 1 through 4, wherein the measuring means determines a gel particle number Xt per time t from the equation

$$X = \Sigma (\omega k \cdot Pk)$$

(where $\omega k$ is a weighting coefficient for particles having a scattering intensity Pk).

6. A sample cell used in the gelation-reaction measuring apparatus according to claims 1 through 5, comprising a container which is sealed by a sealing member and which previously contains therein a reagent that gelates with the target substance to be measured, and a stirring means for stirring an introduced sample and the solution of the reagent.

7. A sample cell according to claim 6, wherein the reagent and the stirring means are sealed in an environment in which the substance to be measured is present at or below a predetermined level.

## FIG. 1

FIG. 2a

FIG. 2b

FIG. 2c

FIG. 2d

## FIG. 3a

ENDOTOXIN (log[pg/ml])

## FIG. 3b

ENDOTOXIN (log [pg/ml])

## FIG. 3c

ENDOTOXIN (log[pg/ml])

## FIG. 4

FIG. 5

FIG. 6

*FIG. 7*

| **INTERNATIONAL SEARCH REPORT** | International application No. |
|---|---|
| | PCT/JP2006/318906 |

A.  CLASSIFICATION OF SUBJECT MATTER
*G01N33/483*(2006.01)i, *G01N15/14*(2006.01)i, *G01N33/579*(2006.01)i

According to International Patent Classification (IPC) or to both national classification and IPC

B.  FIELDS SEARCHED

Minimum documentation searched (classification system followed by classification symbols)
G01N33/483, G01N15/14, G01N33/579

Documentation searched other than minimum documentation to the extent that such documents are included in the fields searched
Jitsuyo Shinan Koho        1922-1996    Jitsuyo Shinan Toroku Koho   1996-2006
Kokai Jitsuyo Shinan Koho  1971-2006    Toroku Jitsuyo Shinan Koho   1994-2006

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X/Y | JP 2003-322655 A  (Daisen Menburen Systems Kabushiki Kaisha), 14 November, 2003 (14.11.03), Claims; Par. Nos. [0026] to [0028] (Family: none) | 1,2/3-7 |
| Y | JP 06-050875 A  (Kowa Co., Ltd.), 25 February, 1994 (25.02.94), Claims; Par. No. [0012]; Fig. 1 & US 5907399 A1        & EP 0582431 A3 | 3-7 |
| A | JP 11-056390 A  (Seikagaku Corp.), 02 March, 1999 (02.03.99), (Family: none) | 1-7 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 October, 2006 (17.10.06) | 24 October, 2006 (24.10.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| International application No. |
|---|
| PCT/JP2006/318906 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 09-159671 A  (Wako Pure Chemical Industries, Ltd.), 20 June, 1997 (20.06.97), (Family: none) | 1-7 |
| A | WO 1995/014932 A1  (Seikagaku Corp.), 01 June, 1995 (01.06.95), & JP 3061418 B          & EP 0731354 A1 | 1-7 |
| A | JP 2005-524075 A  (Biowhitaker Technologies, Inc.), 11 August, 2005 (11.08.05), & WO 2003/093323 A2     & US 2005/0244299 A & EP 1499643 A | 1-7 |
| A | JP 2004-093536 A  (Daisen Menburen Systems Kabushiki Kaisha), 25 March, 2004 (25.03.04), (Family: none) | 1-7 |

Form PCT/ISA/210 (continuation of second sheet) (April 2005)

**EP 2 081 024 A1**

## REFERENCES CITED IN THE DESCRIPTION

### Patent documents cited in the description

- JP 2004093536 A